# EUROPEAN PATENT APPLICATION

(11) **EP 4 417 214 A1**
(43) Date of publication of application: **21.08.2024**
(21) Application number: 22881075.0
(22) Date of filing: 13.10.2022
(51) Int. Cl.: A61K 36/062, A61K 36/064, A61P 31/14, A61P 43/00

(54) **COMPOSITION FOR CORONAVIRUS INFECTION INCLUDING KOJI-DERIVED EXTRACELLULAR VESICLE OR FERMENTED ALCOHOL YEAST¿DERIVED EXTRACELLULAR VESICLE**

(30) Priority: 13.10.2021 JP 2021167835
(71) Applicant: Da Vinci Universale Co., Ltd., Tokyo, 100-0005 (JP); Asahi Shuzo Co., Ltd., Iwakuni-shi, Yamaguchi, 742-0422 (JP)
(72) Inventor: OCHIYA, Takahiro, 1040053 Tokyo (JP); MURANAKA, Asao, 2770832 Chiba (JP)
(74) Representative: J A Kemp LLP
(86) International application number: PCT/JP2022/038145
(87) International publication number: WO 2023/063380

(57) **Abstract**

A pharmaceutical composition for coronavirus infection, comprising a koji-derived extracellular vesicle and/or a fermented sake yeast-derived extracellular vesicle.

## Description

### Technical Field

The present invention relates to a composition for coronavirus infection, comprising a koji-derived extracellular vesicle and/or a fermented sake yeast-derived extracellular vesicle (in particular, a koji-derived exosome).

### Background Art

Exosome is a membrane vesicle secreted from cells, and the surface of an exosome is composed of a lipid bilayer membrane. The exosome is responsible for local or systemic intercellular information transfer by transporting intracellular proteins and genetic information (mRNA, microRNA, etc.) to the outside of the cells. The functions of the exosome are diverse, and it has been reported that, for example, extracellular vesicles derived from Gram-negative bacteria have anti-cancer action (Non Patent Literature 1: Kim OY et al., Nat Commun, 626, 2017).

It has also been suggested that the functions of the exosome are different depending on the cells from which the exosome is derived. For example, when it is an exosome derived from mesenchymal stem cells such as adipose-derived stem cells, the exosome is known to have the function of suppressing inflammation, the function of treating dementia, etc. (Non Patent Literature 2: Katsuda et al., Methods Mol Biol., 1212, 2015).

Thus, it is considered that exosomes secreted from various types of cells can be used to provide various physiological functions and to treat various diseases.

Meanwhile, infectious disease (COVID-19) caused by a highly pathogenic novel coronavirus (SARS-CoV-2) has emerged at the end of 2019, and has been raging in countries over the world since then. Understanding the structure and function of this virus is essential for the development of vaccines and treatment methods against the COVID-19 disease.

In the initial stage of invasion of the novel corona virus into human cells, a protein on the virus surface (a spike protein (S protein)) binds to and adsorb on an angiotensin-converting enzyme II (ACE2) receptor on the human cell surface, resulting in virus invasion and infection (Non Patent Literature 3: Dynamics of the ACE2-SARS-CoV-2/SARS-CoV spike protein interface reveal unique mechanisms. Amanat Ali & Ranjit Vijayan Scientific Reports volume 10, Article number: 14214 (2020)). Moreover, it has been suggested that, in addition to the binding of the S protein to ACE2, the S protein of SARS-CoV-2 virus should bind to CD 147 on the cell surface, and then that it can invade the cells (Non Patent Literature 4: CD147-spike protein is a novel route for SARS-CoV-2 infection to host cells. Wang K et al., Signal Transduction and Targeted Therapy volume 5, Article number: 283 (2020)).

### Citation List

### Non Patent Literature

Non Patent Literature 1: Kim OY et al., Nat Commun, 626, 2017
Non Patent Literature 2: Katsuda et al., Methods Mol Biol., 1212, 2015
Non Patent Literature 3: Amanat Ali & Ranjit Vijayan Scientific Reports volume 10, Article number: 14214 (2020)
Non Patent Literature 4: Wang K et al., Signal Transduction and Targeted Therapy volume 5, Article number: 283 (2020)

### Summary of Invention

### Technical Problem

Under the aforementioned circumstances, it has been desired to develop a substance that inhibits the pathway capable of invading cells and thereby prevents infection.

### Solution to Problem

As a result of intensive studies conducted directed towards achieving the aforementioned object, the present inventor has found that an exosome derived from koji and/or an exosome derived from a fermented sake yeast inhibit the binding of the S protein to an ACE2 receptor or CD147, thereby completing the present invention.

Specifically, the present invention is as follows.
(1) A pharmaceutical composition for coronavirus infection, comprising a koji-derived extracellular vesicle and/or a fermented sake yeast-derived extracellular vesicle.
(2) The pharmaceutical composition according to the above (1), wherein the extracellular vesicle is an exosome.
(3) The pharmaceutical composition according to the above (1) or (2), wherein the koji is rice koji, barley koji or soybean koji.
(4) A binding inhibitor against the binding of a coronavirus spike protein to an angiotensin-converting enzyme II receptor or CD147, wherein the inhibitor comprises a koji-derived extracellular vesicle and/or a fermented sake yeast-derived extracellular vesicle.
(5) The inhibitor according to the above (4), wherein the extracellular vesicle is an exosome.
(6) The inhibitor according to the above (4) or (5), wherein the koji is rice koji, barley koji or soybean koji.

### Advantageous Effects of Invention

According to the present invention, extracellular vesicles that are derived from koji and are derived from a fermented sake yeast are provided. Since the extracellular vesicle of the present invention (in particular, a koji-derived and/or a fermented sake yeast-derived exosome) inhibits the binding of an S protein to an ACE receptor or CD147, the present extracellular vesicle is useful as a pharmaceutical composition for coronavirus infection.

### Brief Description of Drawings

[Figure 1] Figure 1 is a view showing the effects of the extracellular vesicles derived from *Dassai* amazake (rice koji) and derived from a fermented sake yeast to suppress the binding of spike proteins to ACE2 receptors.
[Figure 2] Figure 2 is a view showing the effects of the extracellular vesicles derived from *Dassai* amazake (rice koji) and derived from a fermented sake yeast to suppress the binding of spike proteins to CD147.

### Description of Embodiments

The present invention relates to a pharmaceutical composition for coronavirus infection, comprising a koji-derived extracellular vesicle and/or a fermented sake yeast-derived extracellular vesicle. The composition of the present invention can be used as a pharmaceutical composition for coronavirus infection.

The extracellular vesicle used in the present invention may be any one of an exosome, a microvesicle and an apoptotic body, and it is desirably an exosome. The exosome is an endosome-derived vesicle, the microvesicle is a vesicle directly secreted from cells, and the apoptotic body is a cell fragment generated as a result of cell death.

### 1. Koji-derived extracellular vesicle

The method for producing the koji-derived extracellular vesicle of the present invention will be described below, in particular, taking the exosome as an example. (1) Koji

Koji is made by propagating microorganisms effective for food fermentation, such as koji molds, on grains such as rice, barley, and soybeans. Therefore, the raw materials for koji include rice, barley, and soybeans.

The above-described raw materials for koji are fermented using a koji mold.

The koji mold used in the present invention is a microorganism that has long been used in the brewing of Japanese *sake, miso*, soy sauce, and other products. Since such koji mold has a high ability to secrete and produce proteins, such as several grams of proteins in 1 L of a culture solution, it is also utilized for production of useful proteins such as enzymes.

In the present invention, for example, the following koji molds can be used:
Yellow koji molds: *Aspergillus oryzae, Aspergillus sojae,* etc.;
Black koji molds: *Aspergillus nigar,* etc.;
White koji molds: *Aspergillus kawachii,* etc.; and
Red koji molds: molds belonging to genus Monascus.

### (2) Culture

First, the rice is washed with water until white cloudiness disappears. After discarding the washed water, new water is added, and the rice is soaked until it is completely immersed therein. The time of soaking the rice is not particularly limited, and can be adjusted according to the season. For example, the soaking time is for 3 to 5 hours in summer, for 6 to 12 hours in spring and autumn, and for 15 to 20 hours in winter. After the immersion, the rice is drained in a colander for about 2 to 4 hours, and further, is blended once or twice an hour, so that the top and bottom sides of the rice change. Thereafter, the rice is transferred into a steamer and is then steamed. The steamed rice is quickly spread out on a tray or the like that has been sterilized with alcohol (or *shochu*), and the moisture and the heat after steaming are quickly removed from the surface of the rice by such an action as cutting the rice with a rice scoop or the like.

When the temperature of the steamed rice has reached about 36°C, seed malt is sprinkled on the rice. The amount of the seed malt used is, for example, 2 g of the seed malt for 1 kg of the rice. The rice into which the koji is mixed is quickly gathered together, and is then wrapped with a clean cloth.

The rice wrapped in the cloth is kept warm, such that the temperature thereof becomes 30°C to 32°C. Koji is ready at 45 to 48 hours after the spreading of the seed malt. The state in which koji strongly exhibits a chestnut-like flavor, the mycelium grows, and the rice sticks to one another to form a plate, but in which it can be easily loosened, is the approximate state of the finished koji. To this rice koji (raw material), water is added (1 L of water to 100 grams of the rice koji), and is then left at 4°C for 8 to 16 hours to obtain koji water containing extracellular vesicles. Alternatively, leaving the koji overnight at 30°C to 37°C will also produce rice koji containing extracellular vesicles.

### (3) Recovery

Examples of the method of recovering exosomes from cultures (fermented products such as rice koji) may include an ultracentrifugation method, an immunoprecipitation method, an affinity method using magnetic beads, and a density gradient centrifugation method. Commercially available reagents for exosome isolation can also be employed to isolate and recover exosomes.

In the case of the ultracentrifugation method, high-purity exosomes can be obtained, for example, by previously performing centrifugation at a centrifugal force of about 100 g to 10,000 g for several steps, and then performing ultracentrifugation at a centrifugal force of about 120,000 g for 70 minutes. However, those skilled in the art can determine the centrifugation method and conditions, as appropriate.

Whether the recovered exosomes are the intended ones can be confirmed by morphological observation using a transmission electron microscope, the measurement of the number of particles and particle size using a nanoparticle tracking system, a genetic analysis using PCR, an immunological method (ELISA, FACS, etc.), Western blotting, or other methods.

In addition, whether the recovered exosomes are the intended ones can also be confirmed using, as indicators, proteins recognized as exosome markers. For example, CD9, CD63 and the like can be used as markers for detection.

### 2. Fermented sake yeast-derived extracellular vesicle

Fermented sake is sake made by alcoholic fermentation of raw materials with a yeast.

Accordingly, as a fermented sake yeast used in the present invention, a budding yeast (*Saccharomyces cerevisiae*) can be used.

With regard to a method for producing the fermented sake yeast-derived extracellular vesicle of the present invention (culture and recovery), the same production method as the method for producing a koji-derived extracellular vesicle can be applied. Also, in order to confirm whether the recovered exosome is an exosome of interest, the same method as that described above can be applied.
for confirmation of

### 3. Pharmaceutical composition for coronavirus infection and inhibitor

The present invention provides a pharmaceutical composition for coronavirus infection, comprising a koji-derived extracellular vesicle and/or a fermented sake yeast-derived extracellular vesicle, in particular, a koji- or fermented sake yeast-derived exosome. Moreover, the present invention provides a binding inhibitor against the binding of a coronavirus spike protein to an angiotensin-converting enzyme II (ACE2) receptor or CD147, wherein the inhibitor comprises a koji-derived extracellular vesicle and/or a fermented sake yeast-derived extracellular vesicle.

The pharmaceutical composition and inhibitor of the present invention inhibit the binding of a coronavirus S protein to an ACE2 receptor or CD147. As a result, the S protein cannot bind to the ACE2 receptor or CD147 on the human cell surface, and thus, invasion of the virus into the cell can be suppressed.

The target viruses for the use of the pharmaceutical composition of the present invention include SARS coronavirus (SARS-CoV), novel coronavirus (SARS-CoV-2), Middle East respiratory syndrome coronavirus (MERS-CoV), and the like.

The pharmaceutical composition and inhibitor of the present invention may include a variety of pharmaceutically (medically) acceptable carriers depending on the form of use, as long as the present skin composition contains an effective amount of exosome serving as an active ingredient, without losing its activity. For example, commonly used carriers can be applied as diluents, excipients, etc.

Examples of such carriers may include water, a normal saline (PBS, etc.), and various organic solvents. Examples of the organic solvents may include an alcohol (ethanol, etc.) aqueous solution, glycerol, and olive oil. Moreover, various fillers, thickeners, binders, surface-active agents, pigments, fragrances, etc. can be added.

In addition, the pharmaceutical composition and inhibitor of the present invention can be processed in the form of a parenteral preparation such as a liquid agent, a suspending agent, a spray agent, an emulsion, an ointment, an aqueous gel, or in the form of an oral preparation such as a granule, a powder agent, a tablet or a capsule.

Moreover, in order to use the present composition for an injection and the like, it can also be processed in a freeze-dried product or a granulated product, which is dissolved in a normal saline, a suitable buffer solution (e.g. PBS), etc. to prepare a drug solution, immediately before the use.

The composition of the present invention can be used in a form, by a method, and at a dosage, which depend on the therapeutic purpose and patient.

The additive amount and the number of additions are predicted to be different depending on the type of coronavirus, as well as conditions such as the culture conditions and the type of a medium necessary for obtaining exosomes. Therefore, those skilled in the art can determine the usage and the dosage, as appropriate.

For example, as an effective amount of exosomes, the concentration of exosomes in the culture medium is 1 × 10⁹ cells/mL or more, and is preferably 5 µg/mL to 20 µg/mL.

Furthermore, in order for the inhibitor of the present invention to inhibit the binding of a coronavirus S protein to an ACE2 receptor or CD147 on human cells, it is also possible to test the present inhibitor *in vitro,* as described in the Examples below.

The pharmaceutical composition of the present invention may comprise ingredients other than exosomes, depending on its use purpose and dosage form. Examples of the ingredients other than exosomes may include additives commonly used in production of pharmaceutical products, such as a stabilizer, a preservative, a buffer, a pH adjuster, and an excipient. The contents of such ingredients other than exosomes can also be controlled, as appropriate, by those skilled in the art.

### Examples

Hereinafter, the present invention will be more specifically described in the following Examples. However, these Examples are not intended to limit the scope of the present invention.

### [Example 1]

The degree of DEx (a fermented sake yeast-derived exosome) and a rice koji-derived exosome to inhibit the binding of a viral spike protein to ACE2 and CD147 was examined.

The experiment was carried out as follows.

The fermented sake yeast-derived exosome and the rice koji-derived exosome were prepared by the following methods.
(1) First, the exosomes were each centrifuged at 1,000 x G for 20 minutes to remove foreign substances.
(2) The supernatant was filtered through a 0.22-micrometer membrane, and was then centrifuged in a Beckman Coulter ultracentrifuge at 100,000 x G for 1 hour.
(3) The precipitate was dissolved in PBS(-), and was then ultracentrifuged again under the same conditions as those described above.
(4) The precipitate was dissolved in PBS(-), and its content was adjusted to 1 × 10¹¹ particles/ml by measuring the number of particles using nanosight. The resultant was used as a stock solution in the experiment.

Using COVID-19 SPIKE-ACE2 BINDING ASSAY KIT manufactured by RAYBIO, the binding-inhibitory effects of DEx (a fermented sake yeast-derived exosome) and a rice koji (amazake-derived) exosome against the binding of a spike protein on the surface of a coronavirus to an ACE2 receptor on the cell surface were examined according to the following procedures. As a positive control, the exosome of an ACE2 protein-expressing PNT2 cell was used.

(1) 50 µL each of a solution of DEx (a fermented sake yeast-derived exosome) and a rice koji (amazake-derived) exosome solution (wherein each solution containing 0%, 2%, or 10% exosomes) was mixed with 0.7 µL of an ACE2 protein, and the mixed solution was then diluted with 1 x Assay Diluent of the kit to a total amount of 100 µL.
(2) The plate was covered, and was then incubated (with shaking) for 2.5 hours.
(3) The solution was discarded, and 1 x Wash Buffer of the kit was added in an amount of 300 µl/well to the plate, followed by performing washing 4 times.
(4) 100 µL of a goat-anti-ACE2 antibody was added to the resultant, and the obtained mixture was then incubated (with shaking) at room temperature for 1 hour.
(5) The solution was discarded, and 1 x Wash Buffer was added in an amount of 300 µl/well to the plate, followed by performing washing 4 times.
(6) 100 µL of 1 x HRP-conjugated anti-goat IgG was added to the resultant, and the obtained mixture was then incubated (with shaking) at room temperature for 1 hour.
(7) The solution was discarded, and 1 x Wash Buffer was added in an amount of 300 µl/well to the plate, followed by performing washing 4 times.
(8) 100 µL of TMB (tetramethylbenzidine) One-Step Substrate Reagent was added to the resultant, and the obtained mixture was then incubated at room temperature for 10 minutes (shading against light, with shaking).
(9) 50 µL of Stop solution (a reaction stop solution) was added to the resultant, and the absorbance at 450 nm was then measured.

Subsequently, using a COVID-19 SPIKE protein, the binding inhibitory effects of DEx (a fermented sake yeast-derived exosome) and a rice koji (amazake-derived) exosome against the binding of a spike protein on the surface of a coronavirus to the receptor CD147 on the cell surface were examined according to the following procedures.

Colorimetric ELISA for measuring the binding between the immobilized SARS-CoV-2 S protein RBD and a biotinylated human ACE2 protein was used as a basis.

(1) A plate was coated with the SARS-CoV-2 S protein RBD (ACROBiosystems).
(2) 50 µL of a biotinylated human CD147 protein was added to the plate coated with the Spike protein RBD prepared in (1) above, and a reaction was then performed at room temperature for 30 minutes.
(3) A positive control (in this case, the exosome of CD147-positive human colon cancer cells HCT116), or 100 µL each of a solution of DEx (a fermented sake yeast-derived exosome) and a rice koji (amazake-derived) exosome solution (wherein each solution containing 0%, 2%, or 10% exosomes) was added to the reaction mixture, and the thus obtained mixture was then left at room temperature for 30 minutes.
(4) Streptavidin-HRP was added, and then, TMB colorimetric HRP substrate (Funakoshi Co., Ltd.) was added to the reaction mixture.
(5) The inhibition percentage was calculated from the colorimetric quantification value, and was graphed.

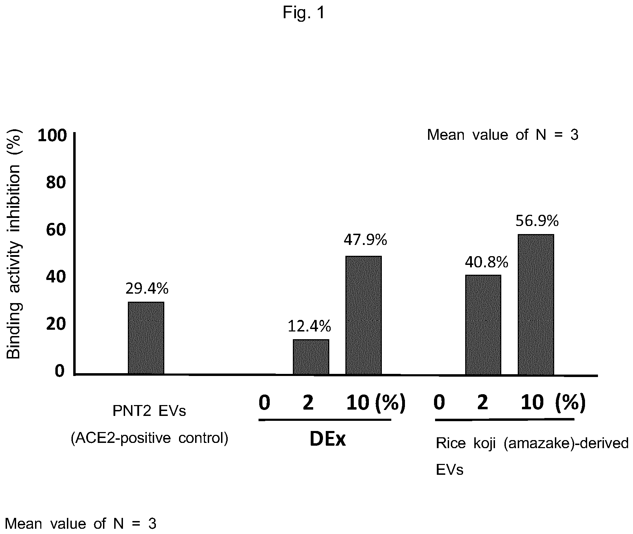

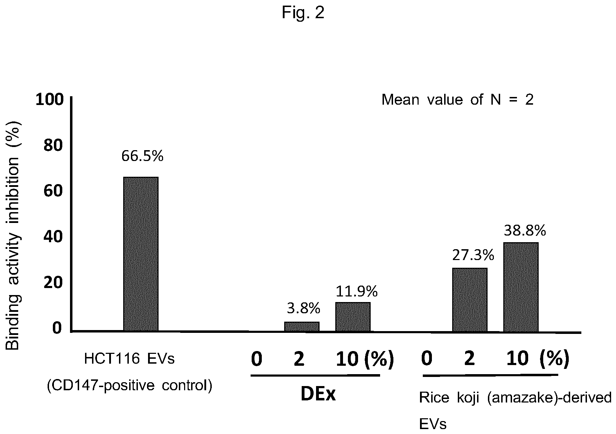

The results are shown in Figures 1 and 2.

From Figures 1 and 2, it was demonstrated that the DEx (a fermented sake yeast-derived exosome) and the rice koji (amazake-derived) exosome have the effect of inhibiting the binding of a novel coronavirus spike protein to ACE2 and CD147. These results show that the DEx (a fermented sake yeast-derived exosome) and the rice koji (amazake-derived) exosome bind, as decoys of the novel coronaviruses, to ACE2 and CD147, so that the DEx and the rice koji (amazake-derived) exosome can competitively inhibit the binding of the novel coronaviruses to the receptors of the novel coronaviruses that are originally present on the cell surface. It is considered that the DEx and the rice koji (amazake-derived) exosome exhibit the effect of preventing the infection with the novel coronavirus, and also exhibits the effect of preventing the spread of the virus in the body in the event of infection.

## Claims

1. A pharmaceutical composition for coronavirus infection, comprising a koji-derived extracellular vesicle and/or a fermented sake yeast-derived extracellular vesicle.

2. The pharmaceutical composition according to claim 1, wherein the extracellular vesicle is an exosome.

3. The pharmaceutical composition according to claim 1 or 2, wherein the koji is rice koji, barley koji or soybean koji.

4. A binding inhibitor against the binding of a coronavirus spike protein to an angiotensin-converting enzyme II receptor or CD147, wherein the inhibitor comprises a koji-derived extracellular vesicle and/or a fermented sake yeast-derived extracellular vesicle.

5. The inhibitor according to claim 4, wherein the extracellular vesicle is an exosome.

6. The inhibitor according to claim 4 or 5, wherein the koji is rice koji, barley koji or soybean koji.
